# FASCICULE DE BREVET EUROPEEN

(11) **EP 1 101 756 B1**
(45) Date de publication et mention de la délivrance du brevet: **19.03.2003**
(21) Numéro de dépôt: 00403106.8
(22) Date de dépôt: 09.11.2000
(51) Int. Cl.: C07D 207/22, C07D 207/14, A61K 31/40, A61P 9/12, A61P 9/00, C07D 209/40

(54) **Nouveaux composés aminopyrroline, leur procédé de préparation et les compositions pharmaceutiques qui les contiennent**
Aminopyrrolidin-Derivate, Verfahren zur ihre Herstellung und diese enthaltende pharmazeutische Zusammensetzungen
Aminopyrrolidine derivatives, processes for their preparation and pharmaceutical compositions comprising them

(30) Priorité: 17.11.1999 FR 9914434
(43) Date de publication de la demande: 23.05.2001
(73) Titulaire: LES LABORATOIRES SERVIER, 92200 Neuilly sur Seine (FR)
(72) Inventeur: Bousquet, Pascal, 67000 Strasbourg (FR); Ehrhardt, Jean-Daniel, 67370 Kleinfrankenheim (FR); Bruban, Véronique, 67000 Strasbourg (FR); Feldman, Josiane, 67000 Strasbourg (FR); Schann, Stephan, 67000 Strasbourg (FR); Scalbert, Elizabeth, 75016 Paris (FR); Pfeiffer, Bruno, 95320 Saint la Leu la Foret (FR); Renard, Pierre, 78150 Le Chesnay (FR)

(56) Documents cités:
- EP-A- 0 155 653
- DE-A- 2 029 297
- S. BUDAVARI (ED.): "The Merck Index, 12 th edition" 1996 , MERCK RESEARCH LABORATORIES, WHITEHOUSE STATION, N.J. USA; ISBN 0911910-12-3 XP002159101 Monograph 1250: Bicuculline

## Description

La présente invention concerne de nouveaux composés aminopyrroline, leur procédé de préparation et les compositions pharmaceutiques qui les contiennent.

Des composés possédant une structure de 2-aminopyrroline ont été décrits pour leurs propriétés antidiarrhéïques (EP 0155653) ou antiparasitaires (DE 2029297).

Les composés de la présente invention possèdent une structure originale caractérisée par la présence d'un groupement cyclopropyle associé au noyau aminopyrroline. Cette structure leur confère des propriétés pharmacologiques intéressantes. En particulier, des tests réalisés ont montré qu'ils étaient capables de réduire la pression artérielle, la fréquence cardiaque, ainsi que les troubles du rythme cardiaque. Les composés de l'invention trouvent donc leurs applications dans le traitement des maladies cardiovasculaires, en particulier de l'hypertension artérielle, de l'arythmie et des troubles associés.

La présente invention concerne les composés de formule (I) : 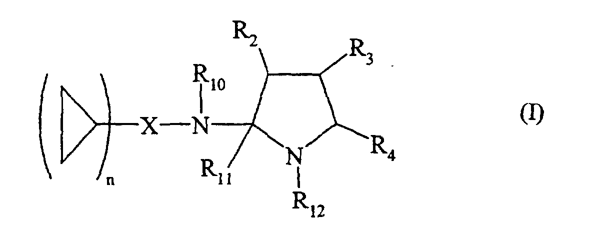 dans laquelle :
- n vaut 1 ou 2,
- X représente un groupement alkylène, alkénylène, alkynylène, ou un groupement arylène éventuellement substitué, ou hétéroarylène éventuellement substitué,
- R₁₀ représente un atome d'hydrogène, ou un groupement alkyle (C₁-C₆) linéaire ou ramifié, et R₁₁ et R₁₂ forment ensemble une liaison, ou bien R₁₂ représente un atome d'hydrogène ou un groupement alkyle (C₁-C₆) linéaire ou ramifié et R₁₀ et R₁₁ forment ensemble une liaison,
- R₂, R₃ et R₄ représentent chacun indépendamment un atome d'hydrogène, un groupement alkyle (C₁-C₆) linéaire ou ramifié, hydroxyalkyle (C₁-C₆) linéaire ou ramifié, alkoxy (C₁-C₆) linéaire ou ramifié, alkoxycarbonyle (C₁-C₆) linéaire ou ramifié, aryle éventuellement substitué, arylalkyle (C₁-C₆) linéaire ou ramifié éventuellement substitué ou aryloxyalkyle (C₁-C₆) linéaire ou ramifié éventuellement substitué, ou bien forment deux à deux avec les atomes de carbone qui les portent un groupement cycloalkyle (C₅-C₇),
étant entendu que :
- le terme alkylène désigne un groupement bivalent linéaire ou ramifié contenant de 1 à 6 atomes de carbone,
- le terme alkénylène désigne un groupement bivalent linéaire ou ramifié contenant de 2 à 6 atomes de carbone et de 1 à 3 doubles liaisons,
- le terme alkynylène désigne un groupement bivalent linéaire ou ramifié contenant de 2 à 6 atomes de carbone et de 1 à 3 triples liaisons,
- le terme aryle représente un groupement phényle ou naphtyle, et le terme arylène représente un groupement bivalent de même type,
- le terme hétéroaryle désigne un groupement mono ou bicyclique insaturé ou partiellement insaturé, comportant de 4 à 11 chaînons et de 1 à 5 hétéroatomes choisis parmi azote, oxygène et soufre, et le terme hétéroarylène représente un groupement bivalent de même type,
- le terme substitué affecté aux expressions aryle, arylène, arylalkyle, aryloxyalkyle, hétéroaryle, hétéroarylène, signifie que les groupements concernés sont substitués sur la partie aromatique par un ou plusieurs atomes d'halogène, ou groupements, identiques ou différents, alkyle (C₁-C₆) linéaire ou ramifié, alkoxy (C₁-C₆) linéaire ou ramifié, hydroxy, cyano, nitro, ou amino (éventuellement substitué par un ou deux groupements alkyle (C₁-C₆) linéaire ou ramifié),
leurs énantiomères, diastéréoisomères, ainsi que leurs sels d'addition à un acide ou à une base pharmaceutiquement acceptable.

Un aspect avantageux de l'invention concerne les composés pour lesquels X représente un groupement arylène éventuellement substitué, ou hétéroarylène éventuellement substitué. Parmi ceux-ci, on préfèrera tout particulièrement ceux pour lesquels X représente un groupement arylène éventuellement substitué, par exemple phénylène.

Un autre aspect avantageux de l'invention concerne les composés pour lesquels X représente un groupement alkylène, alkénylène ou alkynylène, plus particulièrement un groupement alkylène.

Des composés préférés de l'invention sont ceux pour lesquels R₁₁ et R₁₂ forment ensemble une liaison, R₁₀ étant préférentiellement un atome d'hydrogène.

D'autres composés préférés de l'invention sont ceux pour lesquels R₂, R₃ et R₄ représentent chacun un atome d'hydrogène.

Un aspect particulièrement avantageux de l'invention concerne les composés de formule (I) pour lesquels X représente un groupement alkylène, ou un groupement arylène éventuellement substitué, R₁₀ représente un atome d'hydrogène ou un groupement alkyle (C₁-C₆) linéaire ou ramifié, R₁₁ et R₁₂ forment ensemble une liaison, et R₂, R₃ et R₄ représentent chacun indépendamment un atome d'hydrogène, un groupement alkyle (C₁-C₆) linéaire ou ramifié, hydroxyalkyle (C₁-C₆) linéaire ou ramifié, alkoxycarbonyle (C₁-C₆) linéaire ou ramifié, alkoxy (C₁-C₆) linéaire ou ramifié, aryle éventuellement substitué, ou aryloxyalkyle (C₁-C₆) linéaire ou ramifié éventuellement substitué.

Le groupement arylène préféré de l'invention est le groupement phénylène.

Parmi les composés préférés de l'invention, on peut citer plus particulièrement :
- la N-(2-Cyclopropylphényl)-3,4-dihydro-2*H*-pyrrol-5-amine, et ses sels d'addition à un acide pharmaceutiquement acceptable,
- la N-(Dicyclopropylméthyl)-3,4-dihydro-2H-pyrrol-5-amine, et ses sels d'addition à un acide pharmaceutiquement acceptable,
- la N-(2-Cyclopropylphényl)-2-méthyl-3,4-dihydro-2*H*-pyrrol-5-amine, et ses sels d'addition à un acide pharmaceutiquement acceptable.

L'invention concerne également le procédé de préparation des composés de formule (I), caractérisé en ce que l'on utilise comme produit de départ un composé de formule (II) : 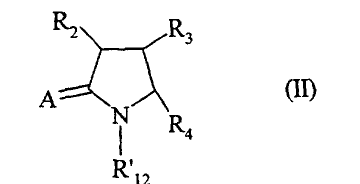 dans laquelle R₂, R₃ et R₄ sont tels que définis dans la formule (I), R'₁₂ représente un atome d'hydrogène ou un groupement alkyle (C₁-C₆) linéaire ou ramifié, et A représente un atome d'oxygène ou de soufre,
qui réagit :
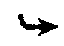 soit avec une amine aromatique de formule (III) : 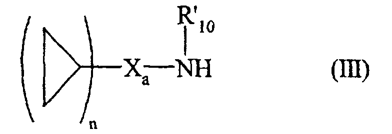 dans laquelle n est tel que défini dans la formule (I), Xₐ représente un groupement arylène ou hétéroarylène, et R'₁₀ représente un atome d'hydrogène ou un groupement alkyle (C₁-C₆) linéaire ou ramifié,
   pour conduire à un composé de formule (I/a) : 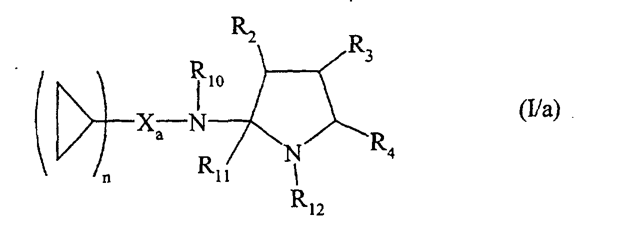 cas particulier des composés de formule (I) pour lequel R₂, R₃, R₄, n sont tels que définis précédemment, Xₐ représente un groupement arylène ou hétéroarylène, et R₁₀, R₁₁ et R₁₂ sont tels que définis dans la formule (I),
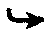 soit avec un agent de méthylation tel que, par exemple, le diméthylsulfate ou l'iodure de méthyle, pour conduire après un traitement en milieu basique à un intermédiaire qui est directement traité en milieu alcoolique par le chlorhydrate d'amine (IV) : 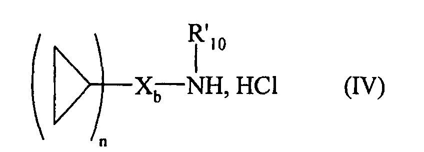 dans laquelle n est tel que défini dans la formule (I), X_{b} représente un groupement alkylène, alkénylène ou alkynylène, et R'₁₀ représente un atome d'hydrogène ou un groupement alkyle (C₁-C₆) linéaire ou ramifié,
   pour conduire à un composé de formule (I/b) : 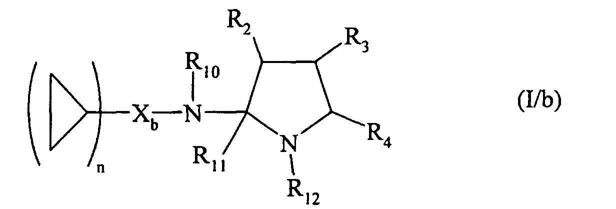 cas particulier des composés de formule (I) pour lequel R₂, R₃, R₄ et n sont tels que définis précédemment, X_{b} représente un groupement alkylène, alkénylène, ou alkynylène, et R₁₀, R₁₁ et R₁₂ sont tels que définis dans la formule (I),
composés (I/a) et (I/b) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

### PREPARATION A : 2-Cyclopropylaniline

Une solution de 5 g (42 mmol) de cyclopropylbenzène dans 20 ml d'anhydride acétique est refroidie à 15°C. Tout en maintenant la température inférieure à 20°C, 3,7 ml d'acide nitrique à 68 % sont ajoutés lentement. Le milieu réactionnel est agité 1 heure à cette température, hydrolysé, alcalinisé par la soude 2N, et extrait 2 fois par 100 ml d'éther. La phase organique est ensuite séchée et concentrée pour conduire à un mélange de 2-nitrocyclopropylbenzène et de 4-nitrocyclopropylbenzène. Le mélange est dissout dans 15 ml d'éthanol et hydrogéné à température et pression normale en présence de 50 mg de PtO₂. Après filtration du catalyseur et évaporation du solvant, le résidu obtenu est purifié par chromatographie sur gel de silice pour conduire au produit du titre.

### PREPARATION B : 4-Cyclopropylaniline

Le produit attendu est obtenu lors de la synthèse du composé décrit dans la préparation A.

### PREPARATION C : 2-Chloro-4-cyclopropylaniline

Une solution de 7,9 mmol (1,05 g) de 4-cyclopropylaniline (préparation B) dans 15 ml d'anhydride acétique est agitée 3 heures à température ambiante. Le milieu réactionnel est concentré. Le résidu est repris dans l'eau, neutralisé, et extrait par 2 fois au dichlorométhane.

La phase organique est séchée et concentrée. Le 4-cyclopropylacétanilide est dissout dans l'acide acétique et traité par 10 mmol de chlore. Après 30 minutes, le solvant est évaporé et le résidu obtenu est repris dans 100 ml d'éthanol puis hydrolysé par de la potasse à reflux. L'éthanol est ensuite évaporé, le résidu repris dans l'eau, et extrait deux fois par du dichlorométhane. La phase organique est ensuite séchée, concentrée et le résidu obtenu est purifié par chromatographie sur gel de silice pour conduire au produit du titre.

### PREPARATION D : 4-Chloro-2-cyclopropylaniline

Le produit attendu est obtenu selon le procédé décrit dans la préparation C en utilisant comme produit de départ le composé décrit dans la préparation A.

### PREPARATION E: 2,4-Dicyclopropylaniline

Le produit attendu est obtenu selon le procédé décrit dans la préparation A partir de m-dicyclopropyl-benzène dont la préparation est décrite dans Chem. Ber., 1973, 106, 511-524.

### PREPARATION F : 2,5-Dicyclopropylaniline

Le produit attendu est obtenu selon le procédé décrit dans la préparation A partir de p-dicyclopropyl-benzène dont la préparation est décrite dans Chem. Ber., 1973, 106, 511-524.

### PREPARATION G : 3,4-Dicyclopropylaniline

Le produit attendu est obtenu selon le procédé décrit dans la préparation A partir de o-dicyclopropyl-benzène dont la préparation est décrite dans Chem. Ber., 1973, 106, 511-524.

### EXEMPLE 1 : N-(2-Cyclopropylphényl)-3,4-dihydro-2H-pyrrol-5-amine, chlorhydrate

A une solution de 5 mmol (666 mg) de 2-cyclopropylaniline dans 30 ml de 1,2-dichloroéthane sont ajoutées 5 mmol (425 mg) de 2-pyrrolidinone et 5 mmol (766 mg) d'oxychlorure de phosphore. L'ensemble est chauffé à 60°C pendant 3 heures 30. Après évaporation du solvant, le résidu est repris dans l'eau, alcalinisé à l'aide de carbonate de sodium et extrait deux fois par 100 ml d'éther. La phase organique est ensuite séchée, concentrée, et purifiée par chromatographie sur gel de silice pour conduire au produit attendu. Le chlorhydrate correspondant est obtenu par action d'une solution titrée d'acide chlorhydrique dans l'étanol.
*Point de fusion :* 182-184°C

### EXEMPLE 2 : N-(4-Chloro-2-cyclopropylphéuyl)-3,4-dihydro-2H-pyrrol-5-amine, chlorhydrate

Le produit attendu est obtenu selon le procédé décrit dans l'exemple 1, en utilisant comme produit de départ le composé décrit dans la préparation D.
*Point de fusion :* 198-199°C

### EXEMPLE 3 : N-(4-Cyclopropylphényl)-3,4-dihydro-2H-pyrrol-5-amine, chlorhydrate

Le produit attendu est obtenu selon le procédé décrit dans l'exemple 1, en utilisant comme produit de départ le composé décrit dans la préparation B.

### EXEMPLE 4 : N-(2-Chloro-4-cyclopropylphényl)-3,4-dihydro-2H-pyrrol-5-amine, chlorhydrate

Le produit attendu est obtenu selon le procédé décrit dans l'exemple 1, en utilisant comme produit de départ le composé décrit dans la préparation C.
*Point de fusion :* 149-151°C

### EXEMPLE 5 : N-(Dicyclopropylméthyl)-3,4-dihydro-2H-pyrrol-5-amine, chlorhydrate

Un mélange de 0,2 mol (25,2 g) de diméthylsulfate et de 0,2 mol (17 g) de 2-pyrrolidinone est chauffé à 60°C pendant une nuit. Le milieu réactionnel est versé dans une solution saturée de K₂CO₃ glacée. La phase aqueuse est extraite deux fois à l'éther et les phases organiques regroupées sont séchées, et concentrées. Le résidu est ensuite distillé sous pression réduite.
2 mmol du produit obtenu sont dissoutes dans 20 ml de méthanol et 2 mmol (295 mg) de chlorhydrate de dicyclopropylméthylamine sont ajoutées. Le milieu réactionnel est agité à 50°C pendant 2 heures. Après évaporation du méthanol, le résidu est repris dans l'eau, alcalinisé et extrait deux fois à l'éther. La phase organique est séchée, concentrée, et purifiée pour conduire au produit attendu. Le chlorhydrate correspondant est obtenu par action d'une solution titrée d'acide chlorhydrique dans l'éthanol.
*Point de fusion :* 223-225°C

### EXEMPLE 6 : N-(Dicyclopropylméthyl)-3-méthyl-3,4-dihydro-2H-pyrrol-5-amine, chlorhydrate

Le produit attendu est obtenu selon le procédé décrit dans l'exemple 5, en remplaçant la 2-pyrrolidinone par la 4-méthyl-2-pyrrolidinone.
*Point de fusion :* 198-199°C

### EXEMPLE 7 : N-(Dicyclopropylméthyl)-2-méthyl-3,4-dihydro-2H-pyrrol-5-amine, chlorhydrate

Le produit attendu est obtenu selon le procédé décrit dans l'exemple 5, en remplaçant la 2-pyrrolidinone par la 5-méthyl-2-pyrrolidinone.
*Point de fusion* : 144-146°C

### EXEMPLE 8 : N-(Dicyclopropylméthyl)-4-méthyl-3,4-dihydro-2H-pyrrol-5-amine, chlorhydrate

Le produit attendu est obtenu selon le procédé décrit dans l'exemple 5, en remplaçant la 2-pyrrolidinone par la 3-méthyl-2-pyrrolidinone.
*Point de fusion :* 201-203°C

### EXEMPLE 9 : N-(Dicyclopropylméthyl)-2,3-diméthyl-3,4-dihydro-2H-pyrrol-5-amine, chlorhydrate

Le produit attendu est obtenu selon le procédé décrit dans l'exemple 5, en remplaçant la 2-pyrrolidinone par la 4,5-diméthyl-2-pyrrolidinone.
*Point de fusion :* 143-145°C

### EXEMPLE 10 : (cis)-N-(Dicyclopropylméthyl)-2,3-diméthyl-3,4-dihydro-2H-pyrrol-5-amine, chlorhydrate

Le produit attendu est obtenu selon le procédé décrit dans l'exemple 5, en remplaçant la 2-pyrrolidinone par la (cis)-4,5-diméthyl-2-pyrrolidinone.
*Point de fusion :* 163-165°C

### EXEMPLE 11 : (trans)-N-(Dicyclopropylméthyl-2,3-diméthyl-3,4-dihydro-2H-pyrrol-5-amine, chlorhydrate

Le produit attendu est obtenu selon le procédé décrit dans l'exemple 5, en remplaçant la 2-pyrrolidinone par la (trans)-4,5-diméthyl-2-pyrrolidinone.
*Point de fusion :* 138-140°C

### EXEMPLE 12 : N-(Dicyclopropylméthyl)-2-éthyl-3,4-dihydro-2H-pyrrol-5-amine, chlorhydrate

Le produit attendu est obtenu selon le procédé décrit dans l'exemple 5, en remplaçant la 2-pyrrolidinone par la 5-éthyl-2-pyrrolidinone.
*Point de fusion :* 108-110°C

### EXEMPLE 13 : 3-(4-Chlorophényl)-N-(dicyclopropylméthyl)-3,4-dihydro-2H-pyrrol-5-amine, chlorhydrate

Le produit attendu est obtenu selon le procédé décrit dans l'exemple 5, en remplaçant la 2-pyrrolidinone par la 4-(4-chlorophényl)-2-pyrrolidinone.
*Point de fusion :* 220-223°C

### EXEMPLE 14 : N-(Dicyclopropylméthyl)-3-[(2-méthylphénoxy)méthyl]-3,4-dihydro-2H-pyrrol-5-amine, chlorhydrate

Le produit attendu est obtenu selon le procédé décrit dans l'exemple 5, en remplaçant la 2-pyrrolidinone par la 4-[(2-méthylphénoxy)méthyl]-2-pyrrolidinone.
*Point de fusion :* 193-194°C

### EXEMPLE 15 : (cis)-N-(dicyclopropylméthyl)-3a,4,5,6,7,7a-hexahydro-3H-indol-2-amine, chlorhydrate

Le produit attendu est obtenu selon le procédé décrit dans l'exemple 5, en remplaçant la 2-pyrrolidinone par la (cis)-octahydro-2*H*-indol-8-one.
*Point de fusion :* 204-206°C

### EXEMPLE 16 : N-(Dicyclopropylméthyl)-4-hydroxyméthyl-3,4-dihydro-2H-pyrrol-5-amine, chlorhydrate

A 10 mmol de 4-hydroxyméthyl-pyrrolidin-thione (dont la préparation est décrite dans Tet. Lett., 1982, 23, 2947-50) en solution dans l'isopropanol sont ajoutées 70 mmoles d'iodure de méthyle. Après agitation à température ambiante, le solvant est évaporé puis de l'éthanol et la dicyclopropylméthylamine (15 mmol) sont ajoutés. Après 24 heures de reflux, le solvant est évaporé, puis le résidu est repris dans de l'eau, alcalinisé puis extrait avec du dichlorométhane. Après lavage, séchage et évaporation de la phase organique, on obtient le produit attendu sous la forme d'un solide blanc.
Le chlorhydrate correspondant est obtenu par action d'une solution titrée d'acide chlorhydrique dans l'éthanol.
*Point de fusion :* 199-200°C

### EXEMPLE 17 : N-(Dicyclopropylméthyl)-4-méthoxycarbonyl-3,4-dihydro-2H-pyrrol-5-amine, chlorhydrate

Le produit attendu est obtenu selon le procédé décrit dans l'exemple 16, à partir de 4-méthoxycarbonyl-pyrrolidin-thione (dont la préparation est décrite dans Tet. Lett., 1982, 23, 2947-50).
*Point de fusion :* 123-125°C

### EXEMPLE 18 : N-(2,4-Dicyclopropylphényl)-3,4-dihydro-2H-pyrrol-5-amine, chlorhydrate

Le produit attendu est obtenu selon le procédé décrit dans l'exemple 1, à partir du composé décrit dans la préparation E.
*Point de fusion :* 163-165°C

### EXEMPLE 19 : N-(2,5-Dicyclopropylphényl)-3,4-dihydro-2H-pyrrol-5-amine, fumarate

Le produit attendu est obtenu selon le procédé décrit dans l'exemple 1, à partir du composé décrit dans la préparation F. Le fumarate est obtenu par action d'une solution titrée d'acide fumarique dans l'éthanol.
*Point de fusion :* 174-176°C

### EXEMPLE 20 : N-(3,4-Dicyclopropylphényl)-3,4-dihydro-2H-pyrrol-5-amine, fumarate

Le produit attendu est obtenu selon le procédé décrit dans l'exemple 19, à partir du composé décrit dans la préparation G.
*Point de fusion :* 202-204°C

### EXEMPLE 21 : N-(2-Cyclopropylphényl)-2-méthyl-3,4-dihydro-2H-pyrrol-5-amine, chlorhydrate

Le produit attendu est obtenu selon le procédé décrit dans l'exemple 1, en remplaçant la 2-pyrrolidinone par la 5-méthyl-2-pyrrolidinone.
*Point de fusion :* 138-139°C

### ETUDE PHARMACOLOGIQUE

### EXEMPLE A : Mise en évidence de l'activité antihypertensive chez le lapin normotendu anesthésié

Les expériences sont réalisées chez le lapin normotendu mâle. Les animaux sont anesthésiés au pentobarbital sodique (40 mg/kg) par la veine marginale de l'oreille. Le lapin est trachéotomisé et immédiatement ventilé à l'air ambiant à l'aide d'un respirateur. Il est ensuite curarisé, et la veine et l'artère fémorales droites sont cathétérisées.

Avant chaque expérience, un volume égal de véhicule est administré et on laisse les paramètres à mesurer se stabiliser pendant 15 à 25 minutes.

Les composés à tester ou le véhicule sont administrés par voie intracisternale (i.c.) ou intraveineuse (i.v.). La pression artérielle (PA) est enregistrée en continu grâce à un cathéter placé dans l'aorte abdominale par l'artère fémorale droite et connecté à un capteur de pression. Les résultats sont exprimés en mmHg. La mesure de la fréquence cardiaque (FC) exprimée en battements par minute (bpm) est effectuée par défilement rapide de l'enregistrement de PA et comptage sur 6 secondes d'enregistrement.
Il apparaît que les composés de l'invention induisent une baisse de la pression artérielle et une bradycardie durables.

A titre d'exemple les résultats obtenus avec le composé de l'exemple 1 sont résumés dans le tableau suivant :

| **Composé de** **l'exemple 1** **Dose (mg/kg)** | **PA** **avant injection** **(mmHg)** | **PA** **après injection** **(mmHg)** | **FC** **avant injection** **(bpm)** | **FC** **après injection** **(bpm)** |
|---|---|---|---|---|
| 1 (i.v.) | 84 | 63 | 306 | 236 |
| 0,3 (i.v.) | 92 | 74 | 300 | 263 |
| 0,03 (i.c.) | 90 | 63 | 307 | 240 |

### EXEMPLE B : Mise en évidence de l'activité antihypertensive chez le rat SH

Les expériences sont réalisées chez le rat mâle spontanément hypertendu (SH). Les animaux sont anesthésiés au pentobarbital par voie intrapéritonéale (45 mg/kg). Les veines et artères fémorales sont cathétérisées pour l'administration des composés à tester. Les animaux sont curarisés et ventilés à l'air ambiant à l'aide d'un respirateur.

Avant chaque expérience, un volume égal de véhicule est administré et on laisse les paramètres à mesurer se stabiliser pendant 15 à 25 minutes.

Les composés à tester ou le véhicule sont administrés par voie intracisternale (i.c.) ou intraveineuse (i.v.). La pression artérielle (PA) est enregistrée en continu grâce à un cathéter placé dans l'aorte abdominale par l'artère fémorale droite et connecté à un capteur de pression. Les résultats sont exprimés en mmHg. La mesure de la fréquence cardiaque (FC) exprimée en battements par minute (bpm) est effectuée par défilement rapide de l'enregistrement de PA et comptage sur 6 secondes d'enregistrement.

Il apparaît que les composés de l'invention induisent une hypotension et une bradycardie durables.

A titre d'exemple, les résultats obtenus avec les composés des exemples 1 et 21 sont résumés dans le tableau suivant :

| **Exemple** | **Dose** **(mg/kg)** | **PA** **avant** **injection** **(mmHg)** | **PA** **après** **injection** **(mmHg)** | **FC** **avant** **injection** **(bpm)** | **FC** **après** **injection** **(bpm)** |
|---|---|---|---|---|---|
| 1 | 1 (i.v.) | 163 | 91 | 386 | 296 |
| 21 | 10 (i.v.) | 166 | 138 | 375 | 320 |
| 21 | 0,1 (i.c.) | 134 | 99 | 345 | 320 |

En outre, le composé de l'exemple 21 est dénué de tout effet vasoconstricteur, et ce quelle que soit la dose utilisée.

### EXEMPLE C : Mise en évidence de l'activité antiarythmique chez le lapin normotendu anesthésié

Les expériences sont réalisées chez le lapin normotendu mâle. Les animaux sont anesthésiés au pentobarbital sodique (40 mg/kg) par la veine marginale de l'oreille. Le lapin est trachéotomisé et immédiatement ventilé à l'air ambiant à l'aide d'un respirateur. Il est ensuite curarisé, et la veine et l'artère fémorales droites sont cathétérisées.

L'enregistrement de l'électrocardiogramme est réalisé par des électrodes transcutanées.

Les composés à tester ou le véhicule, puis la bicuculline (10 µg/kg), sont administrés par voie intracisternale (i.c.).

L'électrocardiogramme est ensuite enregistré en continu pendant 30 min afin de dénombrer les troubles du rythme.

Les résultats sont exprimés en nombre d'extrasystoles ventriculaires (ESV).

Il apparaît que les composés de l'invention induisent une baisse du nombre d'ESV.

A titre d'exemple, les résultats obtenus avec le composé de l'exemple 9 sont résumés dans le tableau suivant :

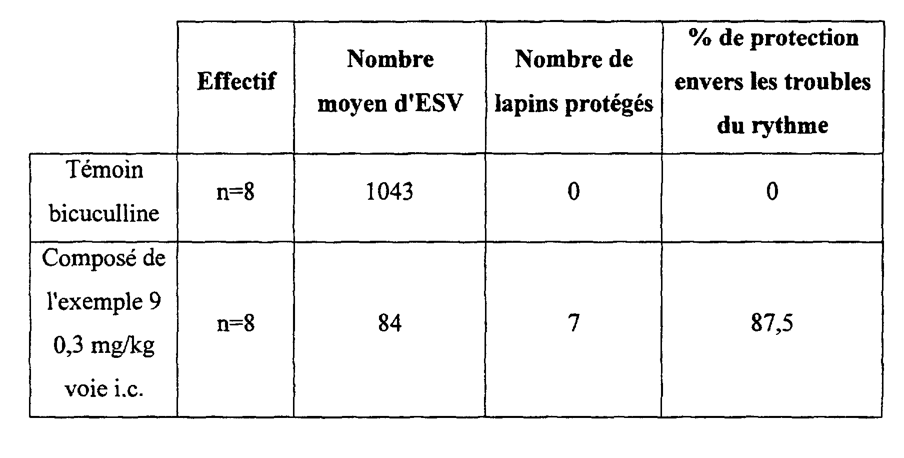

### EXEMPLE D : Composition pharmaceutique

Formule de préparation pour 1000 comprimés dosés à 10 mg :

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 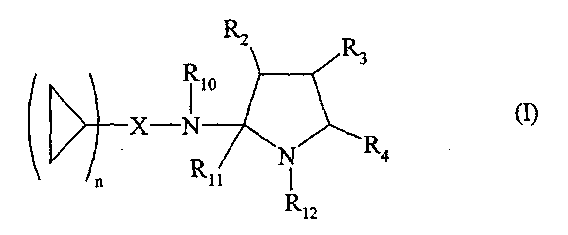 dans laquelle :
• n vaut 1 ou 2,
• X représente un groupement alkylène, alkénylène, alkynylène, ou un groupement arylène éventuellement substitué, ou hétéroarylène éventuellement substitué,
• R₁₀ représente un atome d'hydrogène, ou un groupement alkyle (C₁-C₆) linéaire ou ramifié, et R₁₁ et R₁₂ forment ensemble une liaison, ou bien R₁₂ représente un atome d'hydrogène ou un groupement alkyle (C1-C6) linéaire ou ramifié et R₁₀ et R₁₁ forment ensemble une liaison,
• R₂, R₃ et R₄ représentent chacun indépendamment un atome d'hydrogène, un groupement alkyle (C₁-C₆) linéaire ou ramifié, hydroxyalkyle (C₁-C₆) linéaire ou ramifié, alkoxy (C₁-C₆) linéaire ou ramifié, alkoxycarbonyle (C₁-C₆) linéaire ou ramifié, aryle éventuellement substitué, arylalkyle (C₁-C₆) linéaire ou ramifié éventuellement substitué ou aryloxyalkyle (C₁-C₆) linéaire ou ramifié éventuellement substitué, ou bien forment deux à deux avec les atomes de carbone qui les portent un groupement cycloalkyle (C₅-C₇),
étant entendu que :
- le terme alkylène désigne un groupement bivalent linéaire ou ramifié contenant de 1 à 6 atomes de carbone,
- le terme alkénylène désigne un groupement bivalent linéaire ou ramifié contenant de 2 à 6 atomes de carbone et de 1 à 3 doubles liaisons,
- le terme alkynylène désigne un groupement bivalent linéaire ou ramifié contenant de 2 à 6 atomes de carbone et de 1 à 3 triples liaisons,
- le terme aryle représente un groupement phényle ou naphtyle, et le terme arylène représente un groupement bivalent de même type,
- le terme hétéroaryle désigne un groupement mono ou bicyclique insaturé ou partiellement insaturé, comportant de 4 à 11 chaînons et de 1 à 5 hétéroatomes choisis parmi azote, oxygène et soufre, et le terme hétéroarylène représente un groupement bivalent de même type,
- le terme substitué affecté aux expressions aryle, arylène, arylalkyle, aryloxyalkyle, hétéroaryle, hétéroarylène, signifie que les groupements concernés sont substitués sur la partie aromatique par un ou plusieurs atomes d'halogène, ou groupements, identiques ou différents, alkyle (C₁-C₆) linéaire ou ramifié, alkoxy (C₁-C₆) linéaire ou ramifié, hydroxy, cyano, nitro, ou amino (éventuellement substitué par un ou deux groupements alkyle (C₁-C₆) linéaire ou ramifié),
leurs énantiomères, diastéréoisomères, ainsi que leurs sels d'addition à un acide ou à une base pharmaceutiquement acceptable.

2. Composés de formule (I) selon la revendication 1 pour lesquels X représente un groupement arylène éventuellement substitué, ou hétéroarylène éventuellement substitué, leurs énantiomères, diastéréoisomères ainsi que leurs sels d'addition à un acide ou à une base pharmaceutiquement acceptable.

3. Composés de formule (I) selon la revendication 1 pour lesquels X représente un groupement alkylène, alkénylène ou alkynylène, leurs énantiomères, diastéréoisomères ainsi que leurs sels d'addition à un acide ou à une base pharmaceutiquement acceptable.

4. Composés de formule (I) selon la revendication 1 pour lesquels R₁₁ et R₁₂ forment ensemble une liaison, leurs énantiomères, diastéréoisomères ainsi que leurs sels d'addition à un acide ou à une base pharmaceutiquement acceptable.

5. Composés de formule (I) selon la revendication 1 pour lesquels R₂, R₃ et R₄ représentent chacun un atome d'hydrogène, leurs énantiomères, diastéréoisomères ainsi que leurs sels d'addition à un acide ou à une base pharmaceutiquement acceptable.

6. Composés de formule (I) selon la revendication 1 pour lesquels X représente un groupement alkylène, ou un groupement arylène éventuellement substitué, R₁₀ représente un atome d'hydrogène ou un groupement alkyle (C₁-C₆) linéaire ou ramifié, R₁₁ et R₁₂ forment ensemble une liaison, et R₂, R₃ et R₄ représentent chacun indépendamment un atome d'hydrogène, un groupement alkyle (C₁-C₆) linéaire ou ramifié, hydroxyalkyle (C₁-C₆) linéaire ou ramifié, alkoxycarbonyle (C₁-C₆) linéaire ou ramifié, alkoxy (C₁-C₆) linéaire ou ramifié, aryle éventuellement substitué, ou aryloxyalkyle (C₁-C₆) linéaire ou ramifié éventuellement substitué, leurs énantiomères, diastéréoisomères ainsi que leurs sels d'addition à un acide ou à une base pharmaceutiquement acceptable.

7. Composé de formule (I) selon la revendication 1 qui est la N-(2-cyclopropylphényl)-3,4-dihydro-2*H*-pyrrol-5-amine, ainsi que ses sels d'addition à un acide pharmaceutiquement acceptable.

8. Composé de formule (I) selon la revendication 1 qui est la N-(dicyclopropylméthyl)-3,4-dihydro-2*H*-pyrrol-5-amine, ainsi que ses sels d'addition à un acide pharmaceutiquement acceptable.

9. Composé de formule (I) selon la revendication 1 qui est la N-(2-Cyclopropylphényl)-2-méthyl-3,4-dihydro-2*H*-pyrrol-5-amine, ainsi que ses sels d'addition à un acide pharmaceutiquement acceptable.

10. Procédé de préparation des composés de formule (I) selon la revendication 1 caractérisé en en ce que l'on utilise comme produit de départ un composé de formule (II) : 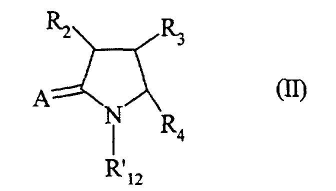 dans laquelle R₂, R₃ et R₄ sont tels que définis dans la formule (I), R'₁₂ représente un atome d'hydrogène ou un groupement alkyle (C₁-C₆) linéaire ou ramifié, et A représente un atome d'oxygène ou de soufre,
qui réagit :
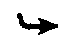 soit avec une amine aromatique de formule (III) : 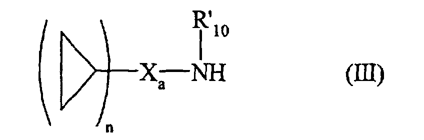 dans laquelle n est tel que défini dans la formule (I), Xₐ représente un groupement arylène ou hétéroarylène, et R'₁₀ représente un atome d'hydrogène ou un groupement alkyle (C₁-C₆) linéaire ou ramifié,
pour conduire à un composé de formule (I/a) : 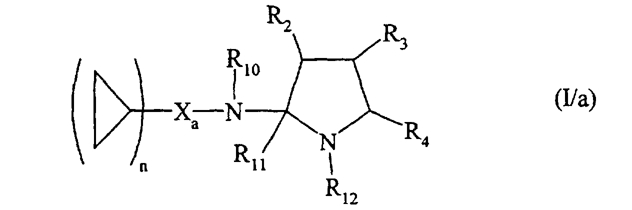 cas particulier des composés de formule (I) pour lequel R₂, R₃, R₄, n sont tels que définis précédemment, Xₐ représente un groupement arylène ou hétéroarylène, et R₁₀, R₁₁ et R₁₂ sont tels que définis dans la formule (I),
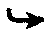 soit avec un agent de méthylation tel que, par exemple, le diméthylsulfate ou l'iodure de méthyle, pour conduire après un traitement en milieu basique à un intermédiaire qui est directement traité en milieu alcoolique par le chlorhydrate d'amine (IV) : 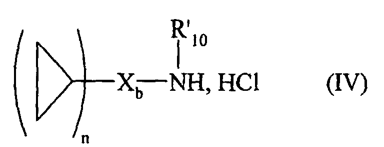 dans laquelle n est tel que défini dans la formule (I), X_{b} représente un groupement alkylène, alkénylène ou alkynylène, et R'₁₀ représente un atome d'hydrogène ou un groupement alkyle (C₁-C₆) linéaire ou ramifié,
pour conduire à un composé de formule (I/b) : 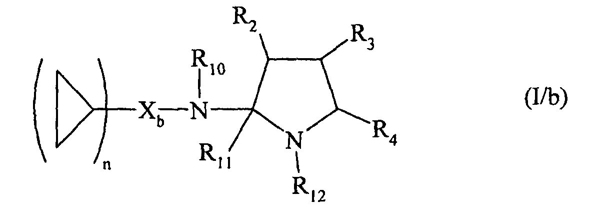 cas particulier des composés de formule (I) pour lequel R₂, R₃, R₄ et n sont tels que définis précédemment, X_{b} représente un groupement alkylène, alkénylène, ou alkynylène, et R₁₀, R₁₁ et R₁₂ sont tels que définis dans la formule (I),
composés (I/a) et (I/b) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

11. Composition pharmaceutique contenant comme principe actif au moins un composé selon l'une quelconque des revendications 1 à 9, seul ou en combinaison avec un ou plusieurs excipients ou véhicules inertes, non toxiques, pharmaceutiquement acceptables.

12. Composition pharmaceutique selon la revendication 11 utile pour la fabrication de médicaments utiles dans le traitement des maladies cardiovasculaires.

13. Composition pharmaceutique selon la revendication 12 utile pour la fabrication de médicaments utiles dans le traitement de l'hypertension artérielle.

14. Composition pharmaceutique selon la revendication 12 utile pour la fabrication de médicaments utiles dans le traitement de l'arythmie.

## Claims

1. Compounds of formula (I): 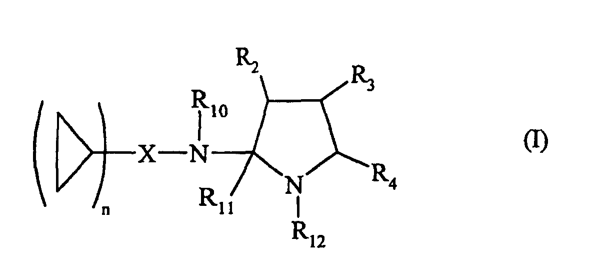 in which:
• n is 1 or 2,
• X represents an alkylene, alkenylene or alkynylene group, or an optionally substituted arylene group, or an optionally substituted heteroarylene group,
• R₁₀ represents a hydrogen atom or a linear or branched (C₁-C₆)alkyl group and R₁₁ and R₁₂ together form a bond, or alternatively R₁₂ represents a hydrogen atom or a linear or branched (C₁-C₆)alkyl group and R₁₀ and R₁₁ together form a bond,
• R₂, R₃ and R₄ each independently of the others represents a hydrogen atom, a linear or branched (C₁-C₆)alkyl group, a linear or branched (C₁-C₆)hydroxyalkyl group, a linear or branched (C₁-C₆)alkoxy group, a linear or branched (C₁-C₆)alkoxycarbonyl group, an optionally substituted aryl group, an optionally substituted arylalkyl group in which the alkyl moiety is linear or branched and has from 1 to 6 carbon atoms, or an optionally substituted aryloxyalkyl group in which the alkyl moiety is linear or branched and has from 1 to 6 carbon atoms, or alternatively two of R₂, R₃ and R₄, with the carbon atoms carrying them, form a (C₅-C₇)cycloalkyl group,
wherein:
- the term alkylene denotes a linear or branched divalent group containing from 1 to 6 carbon atoms,
- the term alkenylene denotes a linear or branched divalent group containing from 2 to 6 carbon atoms and from 1 to 3 double bonds,
- the term alkynylene denotes a linear or branched divalent group containing from 2 to 6 carbon atoms and from 1 to 3 triple bonds,
- the term aryl represents a phenyl or naphthyl group, and the term arylene represents a divalent group of the same type,
- the term heteroaryl denotes a mono- or bi-cyclic unsaturated or partially unsaturated group having from 4 to 11 ring members and from I to 5 hetero atoms selected from nitrogen, oxygen and sulphur, and the term heteroarylene represents a divalent group of the same type,
- the term substituted associated with the expressions aryl, arylene, arylalkyl, aryloxyalkyl, heteroaryl and heteroarylene means that the groups in question are substituted in the aromatic moiety by one or more halogen atoms or identical or different groups linear or branched (C₁-C₆)alkyl, linear or branched (C₁-C₆)alkoxy, hydroxy, cyano, nitro or amino (optionally substituted by one or two linear or branched (C₁-C₆)alkyl groups),
their enantiomers and diastereoisomers, and addition salts thereof with a pharmaceutically acceptable acid or base.

2. Compounds of formula (I) according to claim 1 in which X represents an optionally substituted arylene group or an optionally substituted heteroarylene group, their enantiomers and diastereoisomers, and addition salts thereof with a pharmaceutically acceptable acid or base.

3. Compounds of formula (I) according to claim 1 in which X represents an alkylene, alkenylene or alkynylene group, their enantiomers and diastereoisomers, and addition salts thereof with a pharmaceutically acceptable acid or base.

4. Compounds of formula (I) according to claim 1 in which R₁₁ and R₁₂ together form a bond, their enantiomers and diastereoisomers, and addition salts thereof with a pharmaceutically acceptable acid or base. ,

5. Compounds of formula (I) according to claim 1 in which each of R₂, R₃ and R₄ represents a hydrogen atom, their enantiomers and diastereoisomers, and addition salts thereof with a pharmaceutically acceptable acid or base.

6. Compounds of formula (I) according to claim 1 in which X represents an alkylene group or an optionally substituted arylene group, R₁₀ represents a hydrogen atom or a linear or branched (C₁-C₆)alkyl group, R₁₁ and R₁₂ together form a bond, and R₂, R₃ and R₄ each independently of the others represents a hydrogen atom, a linear or branched (C₁-C₆)alkyl group, a linear or branched (C₁-C₆)hydroxyalkyl group, a linear or branched (C₁-C₆)alkoxycarbonyl group, a linear or branched (C₁-C₆)alkoxy group, an optionally substituted aryl group, or an optionally substituted aryloxyalkyl group in which the alkyl moiety is linear or branched and has from 1 to 6 carbon atoms, their enantiomers and diastereoisomers, and addition salts thereof with a pharmaceutically acceptable acid or base.

7. Compound of formula (I) according to claim 1 which is N-(2-cyclopropylphenyl)-3,4-dihydro-2*H*-pyrrol-5-amine, and its addition salts with a pharmaceutically acceptable acid.

8. Compound of formula (I) according to claim 1 which is N-(dicyclopropylmethyl)-3,4-dihydro-2*H*-pyrrol-5-amine, and its addition salts with a pharmaceutically acceptable acid.

9. Compound of formula (I) according to claim 1 which is N-(2-cyclopropylphenyl)-2-methyl-3,4-dihydro-2*H*-pyrrol-5-amine, and its addition salts with a pharmaceutically acceptable acid.

10. Process for the preparation of compounds of formula (I) according to claim 1, which process is **characterised in that** there is used as starting material a compound of formula (II): 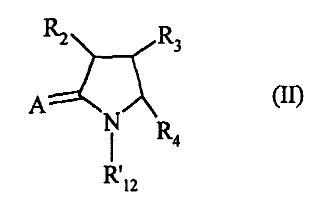 in which R₂, R₃ and R₄ are as defined in formula (I), R'₁₂ represents a hydrogen atom or a linear or branched (C₁-C₆)alkyl group, and A represents an oxygen atom or a sulphur atom,
which is reacted:
⇒ either with an aromatic amine of formula (III): 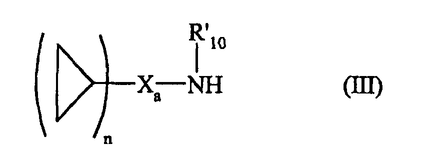 in which n is as defined in formula (I), Xₐ represents an arylene or heteroarylene group, and R'₁₀ represents a hydrogen atom or a linear or branched (C₁-C₆)alkyl group,
to yield a compound of formula (I/a): 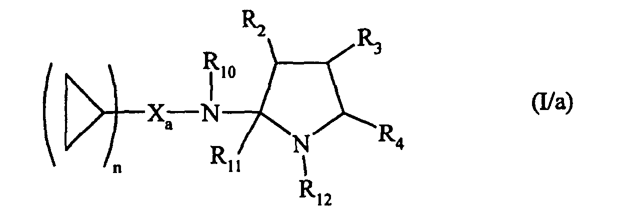 a particular case of the compounds of formula (I) in which R₂, R₃, R₄ and n are as defined above, Xₐ represents an arylene or heteroarylene group, and R₁₀, R₁₁ and R₁₂ are as defined in formula (I),
⇒ or with a methylating agent, such as, for example, dimethyl sulphate or methyl iodide, to yield, after treatment in a basic medium, an intermediate which is treated directly in an alcoholic medium with an amine hydrochloride (IV): 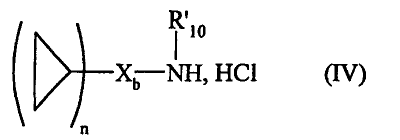 in which n is as defined in formula (I), X_{b} represents an alkylene, alkenylene or alkynylene group, and R'₁₀ represents a hydrogen atom or a linear or branched (C₁-C₆)alkyl group,
to yield a compound of formula (I/b): 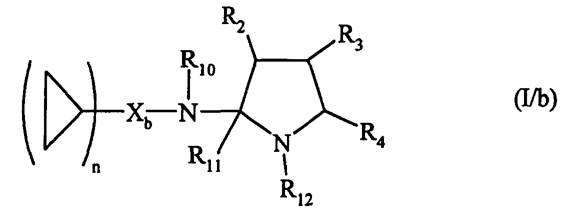 a particular case of the compounds of formula (I) in which R₂, R₃, R₄ and n are as defined above, X_{b} represents an alkylene, alkenylene or alkynylene group, and R₁₀, R₁₁ and R₁₂ are as defined in formula (I),
which compounds (I/a) and (I/b) form the totality of the compounds of formula (I), and:
- which may, where appropriate, be purified according to a conventional purification technique,
- which are separated, where appropriate, into their stereoisomers according to a conventional separation technique,
- which are converted, if desired, into their addition salts with a pharmaceutically acceptable acid or base.

11. Pharmaceutical composition comprising as active ingredient at least one compound according to any one of claims 1 to 9, alone or in combination with one or more inert, non-toxic, pharmaceutically acceptable excipients or carriers.

12. Pharmaceutical composition according to claim 11 for use in the manufacture of medicaments for use in the treatment of cardiovascular diseases.

13. Pharmaceutical composition according to claim 12 for use in the manufacture of medicaments for use in the treatment of arterial hypertension.

14. Pharmaceutical composition according to claim 12 for use in the manufacture of medicaments for use in the treatment of arrhythmia.

## Patentansprüche

1. Verbindungen der Formel (I): 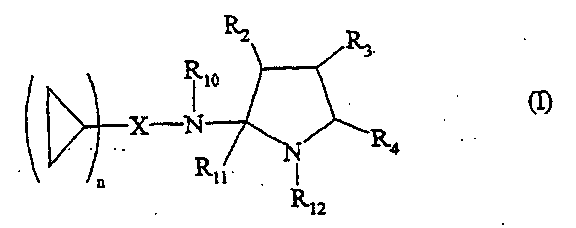 in der:
• n 1 oder 2 bedeutet,
• X eine Alkylen-, Alkenylen-, Alkinylen-Gruppe oder eine gegebenenfalls substituierte Arylengruppe oder gegebenenfalls substituierte Heteroarylengruppe darstellt,
• R₁₀ ein Wasserstoffatom, eine geradkettige oder verzweigte (C₁-C₆)-Alkylgruppe bedeutet und R₁₁ und R₁₂ gemeinsam eine Bindung bilden oder R₁₂ ein Wasserstoffatom oder eine geradkettige oder verzweigte (C₁-C₆)-Alkylgruppe darstellt und R₁₀ und R₁₁ gemeinsam eine Bindung bilden,
• R₂, R₃ und R₄ jeweils unabhängig voneinander ein Wasserstoffatom, eine geradkettige oder verzweigte (C₁-C₆)-Alkylgruppe, geradkettige oder verzweigte (C₁-C₆)-Hydroxyalkylgruppe. geradkettige oder verzweigte (C₁-C₆)-Alkoxygruppe, geradkettige oder verzweigte (C₁-C₆)-Alkoxycarbonylgruppe. gegebenenfalls substituierte Arylgruppe, geradkettige oder verzweigte, gegebenenfalls substituierte Aryl-(C₁-C₆)-alkylgruppe oder eine geradkettige oder verzweigte, gegebenenfalls substituierte Aryloxy-(C₁-C₆)-alkylgruppe bedeuten oder jeweils zwei dieser Gruppen zusammen mit den sie tragenden Kohlenstoffatomen eine (C₅-C₇)-Cycloalkylgruppe bilden,
wobei es sich versteht, daß:
- der Begriff Alkylen eine zweiwertige, geradkettige oder verzweigte Gruppe bedeutet, die 1 bis 6 Kohlenstoffatome enthält,
- der Begriff Alkenylen eine zweiwertige, geradkettige oder verzweigte Gruppe bedeutet, die 2 bis 6 Kohlenstoffatome und 1 bis 3 Doppelbindungen ententhält,
- der Begriff Alkinylen eine zweiwertige, geradkettige oder verzweigte Gruppe bedeutet, die 2 bis 6 Kohlenstoffatome und 1 bis 3 Dreifachbindungen enthält,
- der Begriff Aryl für eine Phenyl- oder Naphthylgruppe und der Begriff Arylen für eine zweiwertige Gruppe der gleichen Art stehen.
- der Begriff Heteroaryl eine mono- oder bicyclische, ungesättigte oder teilweise ungesättigte Gruppe bedeutet, die 4 bis 11 Kettenglieder und 1 bis 5 Heteroatome, ausgewählt aus Stickstoff, Sauerstoff und Schwefel aufweist, und der Begriff Heteroarylen eine zweiwertige Gruppe gleicher Art bedeutet,
- der Begriff substituiert im Hinblick auf die Begriffe Aryl, Arylen, Arylalkyl, Aryloxyalkyl, Heteroaryl und Heteroarylen bedeutet, daß die betroffenen Gruppen am aromatischen Teil durch ein oder mehrere Halogenatome oder gleichartige oder verschiedene, geradkettige oder verzweigte (C₁-C₆)-Alkylgruppen, geradkettige oder verzweigte (C₁-C₆)-Alkoxygruppen, Hydroxygruppen, Cyanogruppen, Nitrogruppen oder (gegebenenfalls durch ein oder zwei geradkettige oder verzweigte (C₁-C₆)-Alkylgruppen substituierte) Aminogruppen substituiert sind,
deren Enantiomere, Diastereoisomere sowie deren Additionssalze mit einer pharmazeutisch annehmbaren Säure oder Base.

2. Verbindungen der Formel (I) nach Anspruch 1, worin X eine gegebenenfalls substituierte Arylengruppe, oder eine gegebenenfalls substituierte Heteroarylengruppe darstellt, deren Enantiomere, Diastereoisomere sowie deren Additionssalze mit einer pharmazeutisch annehmbaren Säure oder Base.

3. Verbindungen der Formel (I) nach Anspruch 1, worin X eine Alkylen-, Alkenylen- oder Alkinylen-Gruppe darstellt, deren Enantiomere, Diastereoisomere sowie deren Additionssalze mit einer pharmazeutisch annehmbaren Säure oder Base.

4. Verbindungen der Formel (I) nach Anspruch 1, worin R₁₁ und R₁₂ gemeinsam eine Bindung bilden, deren Enantiomere, Diastereoisomere sowie deren Additionssalze mit einer pharmazeutisch annehmbaren Säure oder Base.

5. Verbindungen der Formel (I) nach Anspruch 1, worin R₂, R₃ und R₄ jeweils ein Wasserstoffatom bedeuten, deren Enantiomere, Diastereoisomere sowie deren Additionssalze mit einer pharmazeutisch annehmbaren Säure oder Base.

6. Verbindungen der Formel (I) nach Anspruch 1, worin X eine Alkylengruppe oder eine gegebenenfalls substituierte Arylengruppe, R₁₀ ein Wasserstoffatom oder eine geradkettige oder verzweigte (C₁-C₆)-Alkylgruppe bedeuten, R₁₁ und R₁₂ gemeinsam eine Bindung bilden und R₂, R₃ und R₄ jeweils unabhängig voneinander jeweils ein Wasserstoffatom, eine geradkettige oder verzweigte (C₁-C₆)-Alkylgruppe, geradkettige oder verzweigte (C₁-C₆)-Hydroxyalkylgruppe, geradkettige oder verzweigte (C₁-C₆)-Alkoxycarbonylgruppe, geradkettige oder verzweigte (C₁-C₆)-Alk-oxygruppe, gegebenenfalls substituierte Arylgruppe oder eine gegebenenfalls substituierte, geradkettige oder verzweigte Aryloxy-(C₁-C₆)-alkylgruppe bedeuten, deren Enantiomere, Diastereoisomere sowie deren Additionssalze mit einer pharmazeutisch annehmbaren Säure oder Base.

7. Verbindung der Formel (I) nach Anspruch 1, nämlich N-(2-Cyclopropylphenyl)-3,4-dihydro-2*H*-pyrrol-5-amin sowie dessen Additionssalze mit einer pharmazeutisch annehmbaren Säure.

8. Verbindung der Formel (I) nach Anspruch 1, nämlich N-(Dicyclopropylmethyl)-3,4-dihydro-2*H*-pyrrol-5-amin sowie dessen Additionssalze mit einer pharmazeutisch annehmbaren Säure.

9. Verbindung der Formel (I) nach Anspruch 1, nämlich N-(2-Cyclopropylphenyl)-2-methyl-3,4-dihydro-2*H*-pyrrol-5-amin sowie dessen Additionssalze mit einer pharmazeutisch annehmbaren Säure.

10. Verfahren zur Herstellung der Verbindungen der Formel (I) nach Anspruch 1, **dadurch gekennzeichnet, daß** man als Ausgangsprodukt eine Verbindung der Formel (II) verwendet: 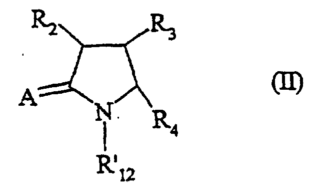 in der R₂, R₃ und R₄ die bezüglich der Formel (I) angegebenen Bedeutungen besitzen, R'₁₂ ein Wasserstoffatom oder eine geradkettige oder verzweigte (C₁-C₆)-Alkylgruppe und A ein Sauerstoffatom oder Schwefelatom bedeuten,
welches man:
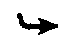 entweder mit einem aromatischen Amin der Formel (III) umsetzt: 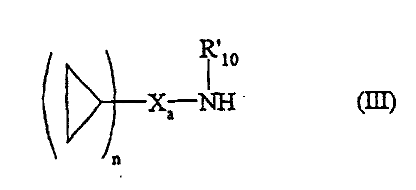 in der n die bezüglich der Formel (I) angegebenen Bedeutungen besitzt, Xₐ eine Arylen- oder Heteroarylen-Gruppe darstellt und R'₁₀ ein Wasserstoffatom oder eine geradkettige oder verzweigte (C₁-C₆)-Alkylgruppe bedeutet,
zur Bildung einer Verbindung der Formel (I/a): 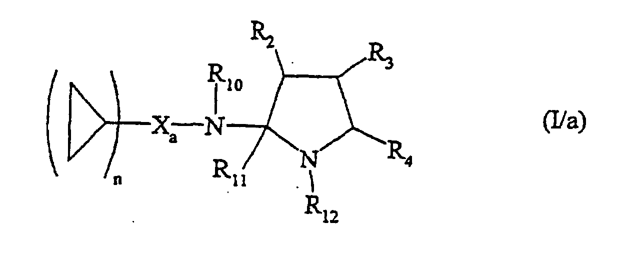 einem Sonderfall der Verbindungen der Formel (I), worin R₂, R₃, R₄ und n die oben angegebenen Bedeutungen besitzen, Xₐ eine Arylen- oder Heteroarylen-Gruppe darstellt und R₁₀, R₁₁ und R₁₂ die bezüglich der Formel (I) angegebenen Bedeutungen besitzen,
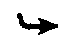 oder mit einem Methylierungsmittel, wie beispielsweise Dimethylsulfat oder Methyliodid umsetzt, so daß man nach einer Behandlung in basischem Medium ein Zwischenprodukt erhält, welches direkt in alkoholischem Medium mit dem Hydrochlorid des Amins (IV) umgesetzt wird: 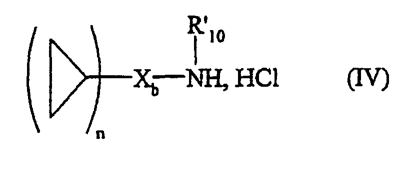 in der n die bezüglich der Formel (I) angegebenen Bedeutungen besitzt, X_{b} eine Alkylen-, Alkenylen- oder Alkinylen-Gruppe darstellt und R'₁₀ ein Wasserstoffatom oder eine geradkettige oder verzweigte (C₁-C₆)-Alkylgruppe bedeutet,
zur Bildung einer Verbindung der Formel (I/b): 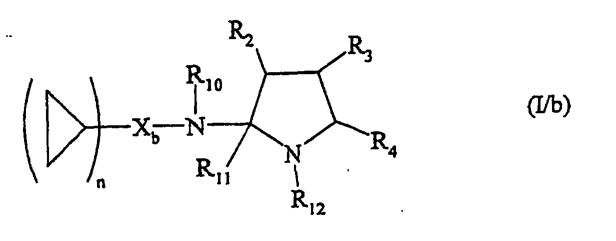 einem Sonderfall der Verbindungen der Formel (I), worin R₂, R₃, R₄ und n die oben angegebenen Bedeutungen besitzen, X_{b} eine Alkylen-, Alkenylenoder Alkinylen-Gruppe darstellt und R₁₀, R₁₁ und R₁₂ die bezüglich der Formel (I) angegebenen Bedeutungen besitzen,
welche Verbindungen (I/a) und (I/b) die Gesamtheit der Verbindungen der Formel (I) bilden, und welche:
- gegebenenfalls mit Hilfe einer klassischen Reinigungsmethode gereinigt werden können,
- gegebenenfalls mit Hilfe einer klassischen Trennmethode in ihre Stereoisomeren getrennt werden, und
- gewünschtenfalls in ihre Additionssalze mit einer pharmazeutisch annehmbaren Säure oder Base umgewandelt werden.

11. Pharmazeutische Zubereitung enthaltend als Wirkstoff mindestens eine Verbindung nach einem der Ansprüche 1 bis 9 allein oder in Kombination mit einem oder mehreren inerten, nicht-toxischen, pharmazeutisch annehmbaren Trägermaterialien oder Bindemitteln.

12. Pharmazeutische Zubereitung nach Anspruch 11, nützlich für die Herstellung von Arzneimitteln für die Behandlung von cardiovaskulären Erkrankungen.

13. Pharmazeutische Zubereitung nach Anspruch 12, nützlich für die Herstellung von Arzneimitteln für die Behandlung der arteriellen Hypertension.

14. Pharmazeutische Zubereitung nach Anspruch 12, nützlich für die Herstellung von Arzneimitteln für die Behandlung von Arhythmien.
